# EUROPEAN PATENT SPECIFICATION

(11) **EP 1 360 491 B1**
(45) Date of publication and mention of the grant of the patent: **11.04.2007**
(21) Application number: 02711080.8
(22) Date of filing: 13.02.2002
(51) Int. Cl.: G01N 33/543

(54) **BIOCHEMICAL METHOD AND APPARATUS FOR DETECTING PROTEIN CHARACTERISTICS**
BIOCHEMISCHES VERFAHREN UND VORRICHTUNG ZUR BESTIMMUNG VON EIGENSCHAFTEN VON PROTEINEN
PROCEDE ET APPAREIL BIOCHIMIQUES DE DETECTION DE CARACTERISTIQUES DE PROTEINES

(30) Priority: 13.02.2001 GB 0103464; 13.02.2001 GB 0103473; 20.02.2001 GB 0104125
(43) Date of publication of application: 12.11.2003
(73) Proprietor: Pronostics Limited, Babraham Cambridge CB2 4AT (GB)
(72) Inventor: GAREY, Caroline, Cambridge CB4 1BN (GB); HADLEY, Jodie, Ely CB6 3WL (GB); ENGLAND, Mark, Cambridge CB4 6DG (GB)
(74) Representative: Sutcliffe, Nicholas Robert
(86) International application number: PCT/GB2002/000628
(87) International publication number: WO 2002/065123

(56) References cited:
- EP-A- 0 126 450
- WO-A-00/55363
- WO-A-01/44812
- WO-A-95/32425
- WO-A-97/14028
- DE-A- 10 031 028
- US-A- 4 568 630
- US-A- 5 206 143
- US-A- 5 519 200
- US-A- 5 981 180
- US-A- 6 096 496

## Description

### Field of the Invention

This invention relates to a biochemical method of detecting protein characteristics according to the preamble of appended Claim. I, and also to an apparatus for detecting protein characteristics according to the preamble of appended Claim 16.

### Background to the Invention

During recent years, there has arisen a considerable interest in techniques and associated systems for determining protein characteristics of numerous types of organisms, for example, yeast, bacteria and mammals as well as cell lines. Currently, tests for detecting protein characteristics require a large number of experimental steps done in a sequential manner. The steps include two-dimensional gel electrophoresis (2D gels), post-electrophoresis extraction of the proteins followed by mass spectrophotometry. The methods for protein analysis are evolving towards greater automation with associated higher detection throughput. Such technological developments have been prompted by, for example, the human genome project; this project has indicated that there are actually in the order of 30, 000 to 40, 000 genes in the human genome. With the discovery of new genetic characteristics there has also been an increased interest in understanding how/why different proteins are produced and function. With millions of protein characteristics and thousands of different specimens to be analysed, high throughput methods of analysing protein characteristics have become very important for the continued progress of protein science, There is, for example, currently a need for massively parallel high throughput technologies for identification and characterisation of proteins (proteomics) relating to drug research and development,

In contrast to genes, proteins cannot be amplified in vitro and therefore only tiny amounts are available for analysis. Thus, the development of protein analysis methods providing better sensitivity and throughput is of utmost significance in making efficient use of protein sequence databases. Despite this need for new high throughput technologies few methods of determining protein characteristics have become commercially available. Current methods are not capable of achieving the throughput and reaction environment required for the detection of protein characteristics.

The techniques used for determining protein characteristics involve a plurality of constituent experiments which are individually labelled; when the experiments have been completed, they can be read using their associated labels for identification. Labels used at present include:
(a) the position of each experiment in a microtitre plate, in a tube, on a 2D gel or injected into a mass spectrophotometer;
(b) the position of each experiment on the surface of a membrane;
(c) the position of each experiment on the surface of a test integrated circuit, known as a "protein array"; and
(d) fluorescent spectrum or other methods of identifying particles to which the experiments are bound.

Such known methods have the disadvantage of employing components for their execution which are difficult and expensive to manufacture and use.

As mentioned above, the main approach for detecting protein characteristics is two-dimensional (2D) gels. This method has the disadvantage as discussed previously of being difficult to analyse, poor reproducibility, variability of results, and limited sample throughput. The two-dimensional gels are very labour intensive to use and often result in only a 50% success rate in protein characterisation. Additional disadvantages for 2D gels are that only a few simultaneous experiments can be performed and additional processing by mass-spectrometry is required before attaining experimental results.

Currently there is limited commercial availability of protein microarrays and most researchers use a 'homebrew' method of analysing proteins, e.g, making an array by attaching a number of proteins to a microscope slide. This approach, combined with methods such as 2D gels and mass spectrophotometry, are likely to prevail unless an alternative method becomes commercially available.

In a United States patent no. US 6, 329, 209 assigned to Zyomyx Inc., a protein microarray is described, The microarray is used for the simultaneous detection of a plurality of proteins which are the expression products, or fragments thereof, of a cell or population of cells in an organism. The microarray concerns a substrate whose surface is partitioned into a plurality of spaced apart immobilisation regions having a plurality of protein-capture agents therein. Each immobilisation region is surrounded by borders which resist non-specific protein binding. Each site is effective labelled by virtue of its spatial position on the surface of the substrate. The protein profiling biochip from Zymomyx Inc. is capable of analysing in the order of 1200 proteins by parallel analysis. While the processing steps of protein analysis may be streamlined with this particular array compared to traditional methods, it does not necessarily offer an increase in throughput as many 2D gels can separate up to a thousand proteins.

As the number of samples tested on the same microarray increases, the demand for associated manufacturing equipment miniaturization and specialized materials handling will render the fabrication of such microarrays increasingly complex The protein characteristics of samples being monitored on such microarrays must be known and isolated beforehand; such prior knowledge makes it a complicated and costly process to manufacture specific microarrays to customer requirements for each different type of organism or species to be studied.

In addition, the amount of interaction between a large volume of proteins or peptide fragments immobilised on the same surface is not well understood and may prevent adequate binding with the test sample. This is problematic as it may therefore decrease the sensitivity and/or specificity of the experiment as well as possibly requiring an increased amount of protein samples in the assay. Further disadvantages associated with this technology are low flexibility, poor reaction kinetics, long manufacturing turnaround times, advanced reader technology, high cost and low data quality.

Bioassays conducted on micro-particles provide another type of massively parallel array technology. Methods of mutually separating different samples have been achieved by attaching information molecules to small supports so that many tests can be performed simultaneously. A system used to mutually distinguish the supports is normally fluorescence or reflection indexes.

Luminex Corporation and Upstate Biotechnology provide a method for detecting serine/threonine kinases by utilising myelin basic protein (MBP) linked to a fluorescent set of supports. The solid supports are microparticles and use optical labels to react with the MBP to indicate an associated reaction. Advanced sorting apparatus is then used to sort out the supports that have reacted, such sorting achieved by way of differential optical signal intensity associated with the different supports; the optical labels are light emitting and the microparticles supports are sorted depending on the intensity of the light emitted therefrom. Such a sorting method allows greater flexibility than microarrays in the detection of protein characteristics through the use of differently loaded microparticles. However, there are still problems experienced concerning the complexity of instrumentation required for determining the different intensity levels of light emitted from the activated microparticles. The throughput of this technology is also currently limited to 100 samples which does not provide the level of multiplexing required for high volume experiments.

In published international PCT application no. WO 00/16893, there is described a method concerning the use of solid supports in a bioassay, and a process for manufacturing such supports. The supports are fabricated from an anodised metal, preferably aluminium. The supports have, for example, antibodies attached thereto for bonding to antigens.

WO-A-97/14028 and US-A-5,981,180 disclose a method for the multiplexed diagnostic and genetic analysis of enzymes, DNA fragments, antibodies, and other biomolecules. Flow cytometry is used to classify labelled beads within a beadset.

WO-A-00/55363 discloses a method of detecting and analysing differences between nucleic acids from two sources, wherein target beads are mixed with two nucleic acids and analysed by flow cytometry.

EP-A-0126450 discloses a method for the detection of antigens or antibodies in a fluid wherein populations of particles can be distinguished from one another by a fluorescent substance, the quantity of that substance, and/ particle size.

US-A-5,206,143 discloses a method for distinguishing multiple subpopulations of biological particles in a sample based upon differences in the fluorescence intensity attributable to one or two fluorochromes with which the biological particles are labelled.

DE-A-10031028 discloses a method for selecting particles with a predetermined characteristic from a population comprising a large number of different particles. WO-A-95/32425 discloses a method for encoding combinatorial libraries using fluorophore labeled beads, and US-A-6,096,496 discloses a combinatorial chemistry bead that includes an electromagnetic spectral emitter as a unique identifier. US-A-4568630 discloses a method of preparing an anodized aluminum printing plate.

US-A-5,519,200 discloses an identification device including a magneto-optical strip and an optical bar code, wherein the magneto-optical strip is positioned in a known location relative to the optical bar code such that on visual identification of the bar code the location of the magneto-optical strip can be found.

Another known application for characterisation and identification of proteins is the analysis of analytes associated with disease. The analysis of the analytes is currently performed using a variety of methods including ELISA (enzyme-linked immunosorbant assay), RIA (radioimmunoassay), chromogenie assays, HPLC (high performance liquid assay), RIA (radioimmunoassay), chromogenic assays, HPLC (high performance liquid chromatography), GCMS (gas chromatography-mass spectroscopy), TLC (thin layer chromatography), NIR (near infrared) analysis. These methods have the disadvantage of being limited in the number of analytes that can be tested at any one time, time-consuming and require expensive equipment.

Another problem experienced with contemporary protein characterization technology is the need for staff to be highly trained and to understand several different system set-ups required when performing increasing numbers of experiments for determining protein characteristics. Such staff requirements results in relatively large initial investments in staff training. It is often necessary, on account of validation requirements and to increase reliability of analysis results, to run experiments repetitively requiring supervision by scientists, which reduces the availability of these scientists for other activities. Moreover, in many industries, such as drug research and development, there are wide ranges of technologies used throughout the process that must all be validated resulting in considerable time requirements and costs.

### Summary of the Invention

A first object of the invention is to provide an improved method of detecting protein characteristics.

A second object of the invention is to provide a low cost high-throughput method of performing experiments for detecting protein characteristics.

A further object of the invention is to provide an improved apparatus for detecting protein characteristics.

According to a first aspect of the invention, in order to address one or more of the aforesaid objects of the invention and other objects that will appear from the following specification, there is provided a method as defined in the accompanying Claim 1.

Moreover, according to a second aspect of the present invention, in order to address one or more of the aforesaid objects of the invention and other objects that will appear from the following specification, there is provided an apparatus as defined in the accompanying Claim 16.

The method and apparatus are of advantage in that they are capable of addressing the aforesaid objects of the invention.

Thus, the first aspect of the present invention concerns a method for detecting protein characteristics, where supports with specific bar codes have an information molecule attached to a main surface thereof. Attaching the molecules onto the supports and suspending them in a fluid allows for very good reaction kinetics, thereby improving sensitivity as well as reducing the reaction volume and time. The sample potentially containing a protein characteristic being detected is added to the fluid. A multiplexed experiment of hundreds of thousands of tests in one is possible since a large number of supports with different bar codes and attached information molecules can be present in the bioassay simultaneously. Use of such molecules in combination with supports decreases the need to perform batched or repeated experiments. Different types of signals are used to indicate the bar codes of the supports and the interaction signal indicating interaction with one or more protein characteristics. Such an approach results in less advanced reader and detector units being required for performing assay measurements, thereby potentially reducing cost.

In a preferred embodiment of the invention, the supports are oxidised prior to the attachment of information molecules thereto. Such attachment allows the surface of the supports to have improved mechanical and chemical attachment properties. Alternatively, or additionally, the supports are coated in one or more molecular binding agents to enhance information molecule attachment thereto.

In a further preferred embodiment of the invention, a measuring unit performs the detection of signal emitting labels and the reading of the bar code substantially simultaneously. This simultaneous measurement decreases the risk of incorrect readings and increases the throughput as advanced software is not employed for the tracking of the supports.

In an additional embodiment of the invention, the reading of the bar code includes locating one or more features arranged to indicate how to interpret the information gathered. This makes it possible to identify the supports irrespectively of their position or flow direction through, for example, a flow cytometer reader system.

A further embodiment of the invention has the fluid including loaded supports placed on and subsequently affixed onto a substrate. This allows a multiple increase of the throughput capacity of the standard planar reading methods while only requiring minor adjustments to existing equipment set ups.

According to a special approach of the invention, the measuring unit's reading involves conveying the substrate with its associated supports along a predetermined path. Such motion along the path is preferably achieved by moving the substrate with supports located thereon while the measuring unit is stationary. It is apparent that, alternatively, the measuring unit could be moved while the substrate with supports is stationary. Such approaches are capable of resulting in substantially all supports in the fluid being analysed. Those supports that are only partially in the measuring unit's focal area along the measuring path have their corresponding positions register so that they are only analysed once.

In other preferred embodiments of the invention, the protein characteristics detected are for drug targeting, proteomics or analysis of analytes. These embodiments of the invention include a system for carrying out massively parallel multiple bioassay tests for drug targeting, proteomics and/or analysis of analytes in a low-cost, fast and convenient manner, Such a scheme achieves high throughput by making a suspension including many thousands of different types of, for example, micro-machined coded supports, also called labels or micro-labels. Bach of these supports carries e.g. nucleic acid, peptide nucleic acid (PNA), enzyme and/or protein information molecules. The supports with attached information molecules are mixed with the sample potentially including the protein characteristic under test together with a signal emitting label, namely a reporter system such as fluorescence. Only supports with nucleic acids, PNAs, enzymes and/or proteins probes that bind to the protein characteristics investigated will bind to the signal emitting label which then emits a signal, for example fluoresce.

In the second aspect of the invention, there is provided an apparatus for detecting protein characteristics, which has detecting means and identifying means arranged to register two different types of signals, the first signal being associated with the detection of activated signal emitting labels and the second signal being associated with the reading of bar codes of supports. Such plurality of different types of signal decreases the potential requirement of using advanced and costly image processing equipment.

An embodiment of a solid support suitably used with the apparatus in a drug targeting, proteomics or analysis of analytes biochemical assay, is substantially linear or planar in shape and has an anodised metal surface layer. The largest dimension of the support is preferably less than circa 250 µm, more preferably less than 150 µm, and most preferably less than circa 100 µm in length, whereby an aqueous suspension is formable from a plurality of the supports. This allows the same type of bioassay to be used for several different experiment types.

In further embodiments, the support's surface layer has a cellular-structure anodisation layer with the growth direction of the cells of the anodisation layer being perpendicular to the plane of the surface layer. Suitably the support has nucleic acid, PNA, enzyme and/or protein information molecules (probe) bound to the surface layer. The support's surface layer may be of aluminium and may also be porous. Further-more the pore size of the surface layer is suitably approximately matched to the size of the nucleic acid, PNA, enzyme and/or protein molecules to be bound. This provides the support with excellent mechanical and chemical bonding properties for the attachment of information molecules.

The bar code incorporated in the support is a spatially varying pattern for identification purposes. Suitably a measuring unit, for example an optical reader, is used for reading the patterns and identifying the supports.

It will be appreciated that features of the invention described in the foregoing can be combined in any combination without departing from the scope of the invention.

### Description of the Drawings

Embodiments of the invention will now be described, by way of example only, with reference to the accompanying drawings wherein:
- Figure 1: is a plan view and a side view of a single support comprising a bar code;
- Figure 2: is a schematic diagram of a bioassay comprising supports, information molecules and signal emitting labels;
- Figure 3: is a cross sectional view in the flow direction of a flow-based reader;
- Figure 4: is a schematic flow diagram of the incubation and reading process of a planar-based reader;
- Figure 5: is a schematic diagram illustrating a planar-based reader for interrogating supports on a planar substrate; and
- Figures 6a, 6b: are schematic top views of a planar substrate illustrating examples of the measuring path taken by the planar-based reader.

### Description of Embodiments of the Invention

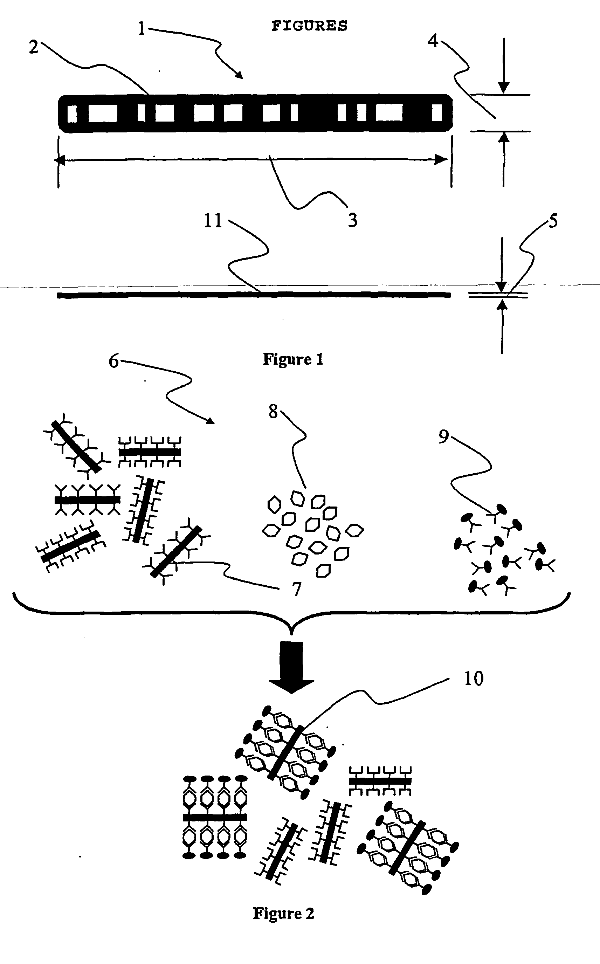

In Figure 1, an illustration of a preferred single support 1 is provided. Such a support will also be referred to as a "micro label" in the following description. The support 1 can be fabricated from a wide variety of materials ranging from polymers, glasses to metal alloys, but is preferably fabricated from a metal and most preferably fabricated from aluminium. The support 1 incorporates a bar code 2 which can be in the shape of at least one (or any combination thereof) of grooves, notches, depressions, protrusions, projections, and most preferably holes. The bar code 2 is suitably a transmission optical bar-code. The bar code 2 is implemented as a spatially sequential series of holes extending through the support 1. Such holes can be of varied shape and size. They are also capable of providing a very good optical contrast as solid areas of the support 1 are substantially non-transmissive to light whereas holes of the bar code 2 are highly transmissive to light received thereat.

The support 1 can be of many different types of shape, but has preferably a substantially planar form with at least a principal surface 11. Each support 1 of this type has a largest dimension 3 of less than circa 250 µm, more preferably less than 150 µm, and most preferably less than circa 100 µm in length. The support 1 has suitably a width 4 to length 3 ratio in a range of circa 1:2 to circa 1:20, although a ratio range of circa 1:5 to circa 1:15 is especially preferred. Moreover, the support I has a thickness 5 which is preferably less than circa 3 µm, and more preferably less than circa 1µm. The thickness of less than circa 1 µm has been shown to provide sufficient mechanical support strength for rendering the support I useable in bioassays. A preferred embodiment of the invention concerns supports 1 having a length 3 of circa 100 µm, a width 4 of circa 10 µm and a thickness 5 of circa 1 µm; such supports are capable of storing up to 100, 000 different identification sequence bar codes 2. Experimental demonstrations of up to 100, 000 different variants of the supports 1 for use in bioassays for protein characterization experiments have been undertaken. Supports 1 of different lengths 3 in a range of 40 to 100 µm carrying between two and five decimal digits of data in the bar code 2, have been fabricated for use in different experiments for the detection of protein characteristics.

Around ten million such supports 1, namely micro-labels, can be fabricated on a single 6-inch diameter substrate, for example a silicon wafer, using contemporary established manufacturing techniques. Conventional photolithography and dry etching processes are examples of such manufacturing techniques used to manufacture and pattern an anodised aluminium layer to yield separate solid supports 1.

A. fabrication process for manufacturing a plurality of supports similar to the support 1 involves the following steps:
(1) depositing a soluble release layer onto a planar substrate;
(2) depositing a layer of aluminium material onto the release layer remote from the substrate;
(3) defining support features in the aluminium material layer by way of photolithographic processes and etching processes;
(4) optionally anodising the aluminium material layer; and
(5) removing the release layer using an appropriate solvent to yield the supports released from the planar substrate.

It will be appreciated that steps (3) and (4) can be executed in either order. Moreover, if required, step (4) can be omitted. Optionally, gaseous anodisation of the aluminium material during step (2) can be employed; such gaseous anodisation is capable of imparting to the supports 1 anodisation regions extending deeply into the supports 1. The release layer is preferably polymethyl methacrylate (PMMA) or other suitable type of material, for example an optical resist as employed in conventional semiconductor microfabrication; the release layer is selected to exhibit properties allowing the aluminium material layer to be held in place with respect to the planar substrate during steps (3) and (4). When PMMA is employed, a suitable solvent comprises acetone and/or methyl isobutyl ketone (MIBK).

Referring now to Figure 2, there is shown a method of detecting protein characteristics in the form of a bioassay indicated generally by 6. The bioassay 6 comprises two binding event experiments denoted by mutually different exposed molecular groupings as illustrated. The assay 6 comprises a plurality of supports, each support being similar to the support 1. Moreover, the assay 6 is generated by mixing together suspensions of chosen sets of active supports 1. Each active support 1 with a corresponding specific bar code 2 has associated therewith a unique information molecule 7, for example a nucleic acid or PNA probe associated therewith, which binds to and/or interacts with a specific type of sample molecule 8 detected during subsequent protein characterization analysis. Information molecules 7 are used in a generic meaning rather then being limited to the meaning of a molecule in its physical or chemical meaning. The information molecules 7 may be attached to the supports 1 either before or after the supports 1 are released from a corresponding planar substrate employed during their fabrication. Enhanced coating of the information molecules 7 onto the supports 1 is achieved by attaching the molecules 7 to the supports 1 after their release from their associated planar manufacturing substrate. Signal emitting labels, for example a label 9, are preferably fluorescent labels. Only supports with information molecules 7 that have bound to the protein characteristic sample molecule 8 detected will fluoresce. The fluorescent label 9 that is bound to the sample molecule 8 detected and indirectly the information molecule 7 causes this fluorescence, denoted by 10. The sample molecule 8 preferably comprises matter for protein characteristic detection. The sample molecule 8 is preferably labelled .with the signal emitting labels 9 before being introduced into the bioassay 6, namely a fluid, preferably a liquid solution and most preferably a liquid solution including water. Alternatively, the signal emitting labels 9 can be introduced into the liquid solution prior to adding the sample to be characterised with respect to proteins. The result of the test is measured by the degree of fluorescence of different types of supports 1. The fluorescent intensity of the signal emitting labels 9 quantifies the level of detected sample molecules 8 with the protein characteristics present in the bioassay 6.

Experiments where a binary yes/no reaction indication is preferred only require determination whether or not the supports 1 in the bioassay 6 are fluorescent.

The information molecules 7 attached to the supports 1 are preferably used in experiments for detecting sample molecules with specific protein characteristics in different embodiments of the invention, for example the molecules 7 can be:
(a) enzyme, protein and/or PNA molecules for analysis of analytes;
(b) nucleic acid, protein and/or PNA molecules for drug targeting; or
(c) nucleic acid, protein and/or PNA molecules for proteomics.

It will be appreciated that the information molecules 7 are not limited to (a) to (c) above and can comprise a broad range of compounds capable of being uniquely distinguished and identified. An example of a suitable compound is a peptide fragment tailored to bind to a specific target. All molecules in this broad range and/or probes may be attached to supports fabricated by steps (1) to (5) above either before or after executing photolithographic operations or releasing the supports I from the planar substrate. The information molecules 7 are preferably attached only to one side of the support 1; alternatively, the molecules 7 preferably cover the support 1 in whole or partially.

The molecules 7 can be arranged to bind only weakly to the supports 1; such weak binding is achieved by arranging for the aluminium surface 11 to be in an untreated state when incubated in a liquid solution, for example an aqueous solution. By modifying the surface 1 of the supports 1 or the information molecules 7, such binding can be selectively enhanced. Anodising the attachment surface 11 of the supports 1 is one way of providing such enhancement. Methods of growing porous surfaces on aluminium are known in the art. Likewise, processes for sealing such porous surfaces are also known. The Applicant has exploited such knowledge to develop a relatively simple process for growing an absorbing surface having accurately controlled porosity and depth. Such porous surfaces are capable of binding well to preferred nucleic acid, PNA, enzyme or protein molecules. Using an avidin-biotin system is another approach for improving binding between the supports 1 and their associated information molecules 7. The support's 1 surface 11 may also be treated with a polymer material such as silane and/or biotin, to further enhance attachment properties. The supports 1 preferably have silane baked onto their surfaces 11. Attaching linking molecules, for example avidin-biotin sandwich system, to the information molecules 7 further enhances their chemical molecular attachment properties.

Such enhanced attachment is important because it allows the probe molecules 7 to be bound strongly to the support surface 11 during manufacture whilst maintaining weak non-specific binding of fluorescent target molecules 8 during tests. Moreover, such enhanced attachment is preferably achieved through having covalent bonds between attachment surface 11 of the support 1 and the information molecule 7. The covalent bonds prevent the information molecules 7 from being dislodged from the supports 1 and causing disturbing background noise in the bioassay 6 during analysis. It is found to be important to wash the active supports 1, said supports having information molecules 7 attached thereto, after attachment to remove any excess information molecules 7 that could otherwise increase the noise in the bioassay 6 during analysis. Discrimination of the tests is thereby enhanced through a better signal-to-noise ratio.

As described in foregoing, each different bar code 2 fabricated onto the supports 1 is associated with a unique corresponding information molecule 7. The bar code 2 is preferably stored on the supports 1 as a series of holes using coding schemes similar to those found on conventional bar code systems, for example as employed for labelling merchandise in commercial retailing outlets. Such a code allows the use of existing reader technology to identify the bar-codes 2 of the supports 1, thereby decreasing the initial investment when adopting technology according to the invention.

Reader systems for use with the bioassay 6 and associated supports will now be described.

The Applicant has developed two classes of reader system. These are based on flow cells for handling the supports 1, and on planar imaging of plated-out supports 1.

A flow-based reader system, similar in construction to a flow cytometer, can be used to draw through thousands of supports 1 per second, thereby reading simultaneously the bar code 2 of each support 1 and the results of its associated test result. The test result is measured as a yes/no binary result or by the degree of fluorescence 10. Alternatively, a planar reader system can be employed, wherein:
(a) the supports 1 are plated out onto a planar substrate; and then
(b) fluorescence microscopy and associated image processing are employed to read the bar codes of the supports and the results of their associated tests.

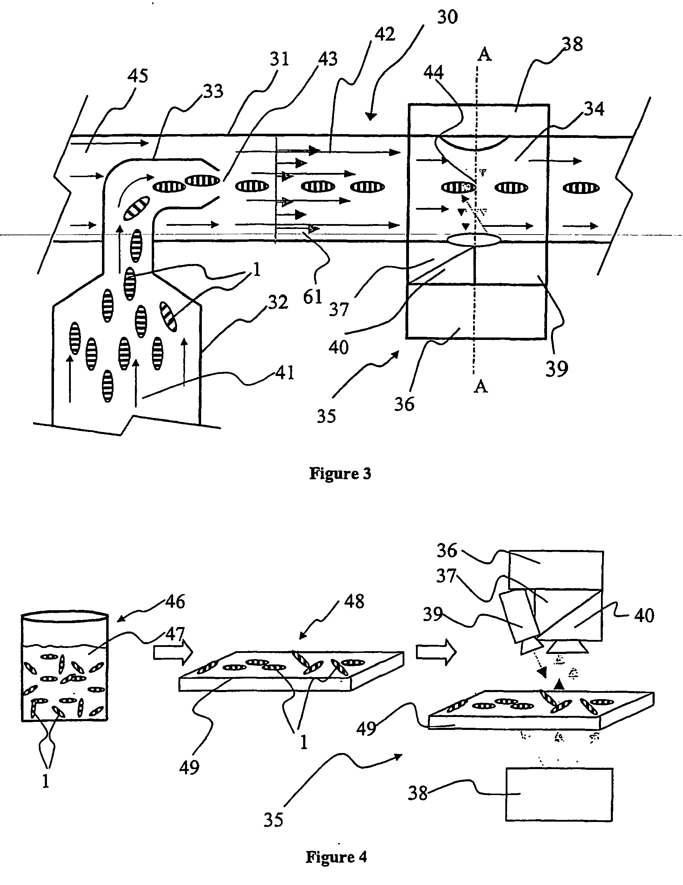

Embodiments of the flow-based reader system and the planar reader system will now be described in further detail with reference to Figures 3,4,5 and 6.

Referring to Figure 3, there is shown a flow-cell reader indicated generally by 30. The reader 30 comprises a flow tube 31 having an upstream end and a downstream end. At the upstream end, there is included within the tube 31 an injection nozzle 33 in fluid communication with an associated focussing zone 32, the zone 32 being situated outside the tube 31. The zone 32 is tapered where it interfaces to the nozzle 33. Moreover, the nozzle 33 comprises at its remote end within the tube 31 an exit aperture 43.

At the downstream end, the reader 30 comprises a measuring unit indicated by 35 for reading supports 1 conveyed in operation in fluid flow from the nozzle 33 at the upstream end to the measuring apparatus 35 at the downstream end. The apparatus 35 includes a reading zone 34, a reader unit 37, a light source 38, a detector unit 40, a signal emitting unit 39 and a processing unit 36. The signal emitting unit 39 is preferably a fluorescent source.

Operation of the reader 30 will be described initially in overview.

A bioassay 6, for example a liquid comprising a plurality of the supports 1 dispersed therein; is introduced into the focussing zone 32. Moreover, a flow of fluid 45, for example filtered water, is generated along the tube 31 in a direction from the upstream end towards the downstream end. Supports 1 in the focussing zone 32 are encouraged, by the tapered profile of the zone 32, to align into a row-like formation as illustrated. The supports 1 are ejected from the exit aperture 43 and are swept in the flow 45 along the tube 31 into the reading zone 34 and eventually therepast. When one or more of the supports 1 enter the reading zone 34, light from the source 38 illuminates the one or more supports 1 so that they appear in silhouette view at the reader unit 37. The reader unit 37 generates a corresponding silhouette signal which is communicated to the processing unit 36 for subsequent image processing to determine the bar code 2 of the supports 1. The signal emitting unit 39 also illuminates the zone 34 with radiation having a wavelength selected to induce fluorescence in one or more the active supports 1. The detector unit 39 detects any fluorescence occurring in the zone 34 and generates a corresponding fluorescence signal which is subsequently received by the processing unit 36. For each support 1 transported through the zone 34, the processing unit 36 is programmed to determine the bar code 2 of the support 1 with its corresponding magnitude of fluorescence. Moreover, the processing unit 36 is also connected to an associated database relating the bar code 2 with a test provided by its associated information molecules 7.

Preferably, the fluid 45 flowing in operation along the tube 31 is a liquid. Alternatively, the fluid 45 can be a gas at reduced pressure relative to the nozzle 33 so that liquid bearing the supports 1 to the exit aperture 43 is vaporised at the aperture 43, thereby assisting to launch supports 1 into the tube 31. Whereas it is easier to establish a laminar flow regime within the tube 31 when fluid flowing therethrough is a liquid, gas flow through the tube 31 potentially offers extremely fast support 1 throughput and associated interrogation in the zone 34.

Design and operation of the reader 30 will now be described in more detail.

The reader 30 is designed to induce the supports 1, namely micro-labels, to flow along a central region of a tube 31 through the defined interrogation zone 34. By utilizing an accelerated sheath fluid 41 configuration and the injecting nozzle 33, the supports 1 injected into the central region of the tube 31 are subjected to a hydrodynamic focusing effect 42 causing all the supports 1 to align lengthwise, namely axially, and to pass through a well-defined focal point 44 in the interrogation zone 34 downstream from an exit aperture 43. In the tube 31, there is a laminar flow of a reading fluid 45 which mixes with the bioassay solution 6 entering the tube 31 through the injection nozzle 33. The distance between the exit aperture 43 and the interrogation zone 34 must be sufficiently long to dissipate any turbulence caused by the injection nozzle 33. This sufficient length allows for a substantially laminar flow of the reading fluid 45 and hence provides the supports 1 with a non-oscillating movement past the focal point 44. If required, the nozzle 33 can be provided with a radially symmetrical arrangement of feed tubelets from the focussing zone 32 so as to obtain a more symmetrical velocity profile within the tube 31. A velocity profile 61 included in Figure 3 provides an illustration of the velocity of the substantially laminar fluid flow in the tube 31; fluid velocity increases from a central region of the tube 31 towards interior peripheral surfaces of the tube 31. In an interface surface region in close proximity to the peripheral surfaces of the tube 31, fluid velocity progressively reduces to substantially zero at the interior surface of the tube 31.

Prior to entering the tube 31, the supports 1 pass through the focusing zone 32 which is operable to arrange the supports 1 for injection into the tube 31. The supports 1 are transported through the tube 31 to the interrogation zone 34 where they are interrogated by the measuring unit 35 when at the focal point 44. Preferably, the supports 1 used in the flow-based reader system 30 have information molecules 7 attached on at least two opposite principal surfaces 11 of the supports 1.

The light source 38 emits light that passes though the reading zone 34 and illuminates the support 1 at the focal point 44. Preferably, the light source 38 emits light in a plane A-A that is substantially perpendicular to the bioassay's flow 45 direction and from two different radial directions, the radial directions preferably having a mutual angle separation, for example with a mutual angular separation of circa 45° separation. Such an arrangement of support 1 illumination in the focal point 44 enables the supports 1 to be identified irrespectively of their rotational position along their longitudinal axis. The reader unit 37, located substantially at an opposite side of the interrogation zone 34 relative to the light source 38, reads the light that passes through one or more supports 1 at the focal point 44. The reader unit 37 is in optical communication with the supports 1 when they pass through the interrogation zone 34. A feature in the form of a marking at one end of each support 1 is used to indicate to the reader unit 37 how to interpret the read information. This allows the support 1 to be read from either direction along its longitudinal axis. The marking is also susceptible to being used to increase the number of possible bar codes on a support 1 to be greatly in excess of 100, 000. For example, employing four different markings on separate sets of supports 1 is capable of increasing the number of identification combinations of supports to about 4.00, 000. An alternative feature to indicate how information codes are to be read is to start each block with 0's and end the blocks with 1's, or vice versa. Further alternatives of these features preferably error checking data, for parity bit checks and/or forward error correction, thereby improving testing reliability.

In operation, the signal emitting unit 39 emits radiation, for example fluorescent light, that causes the supports 1 that have reacted with the sample molecules 8 and the signal emitting label 9 to give off corresponding fluorescent radiation 10. The detector unit 40 measures the magnitude of the intensity of the fluorescent radiation 10 that is given off by the activated signal labels 9 on the supports 1; This intensity indicates the degree of reaction which can be extrapolated to determine the amount of reactive sample molecule 8 present in the protein characteristic bioassay 6 sample. The processing unit 36 then evaluates the information from the detected bar code 2 of the supports 1 measured by the reader unit 37 and to what extent those supports 1 have given off a signal 10 detected by the detector unit 40. The information is then verified with corresponding information in a database comprising preset information linking specific bar code 2 to specific information molecules 7.

Once a sufficient number of supports 1 have been read, the processing unit 36 of the measuring unit 35 calculates the results of the tests associated with the supports 1. This sufficient number is preferably between 10 and 100 copies of each type of supports 1; this number is preferably to enable statistical analysis to be performed on test results. For example, statistical analysis such as mean calculation and standard deviation calculation can be executed for fluorescence 10 associated with each type of information molecule 8 present. The processing unit 36 also controls the reader and detector units 37, 40 so that the each individual support 1 is only analysed once. It could also be possible to only analyse the fluorescent 10 supports 1 that pass through the flow reader 30 to lower the amount of information processed.

In Figure 4, there are shown an incubation process 46 comprising the steps of :
(a) placing supports 1 on a planar .substrate 49, for example a chip, glass slide or microarray, to provide a corresponding support-loaded substrate 48, and
(b) interrogating the support-loaded substrate 48 using a planar measuring unit 35 as illustrated in Figure 3 and described in the foregoing.

The incubation process 46 involves mixing supports 1, bearing attached information molecules 7, with a sample comprising protein characteristic molecules 8 in a liquid bioassay solution 6. The supports 1 are then deposited on the planar substrate 49 and can be subsequently dried to generate the support-loaded substrate 48. Next, the measuring unit 35 measures the level of fluorescence 10 and also the bar code 2 of the different supports 1 of the support-loaded substrate 48. Normally, all the supports 1. on the loaded substrate 48 are analysed to verify the total quality of the experiment In cases where there could be an interest in saving time and/or processing capacity, the software of the processing unit 36 can preferably be configured to analyse only the supports 1 that give off a signal 10, for example through a fluorescent signal label 9, indicating that an interaction with the protein characteristic molecules 8 has occurred. The analysis of the loaded substrate 48 using the planar measuring unit 35 is a very cost effective, easy to perform and suitable way to multiply the analysing capacity for low to medium sample numbers in the range of, for example, single figures to a few thousand supports on each substrate 48.

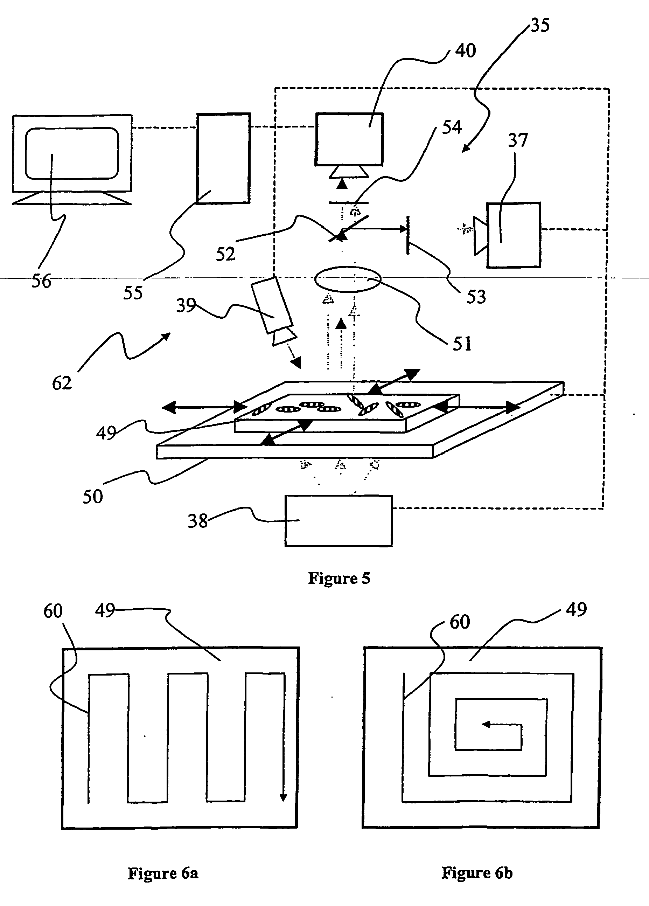

A planar reader system is illustrated in Figure 5 and indicated general by 62. In the reader 62, supports 1 are plated out, namely fixedly deposited or deposited in a liquid, onto the planar light-transmissive substrate 49. Preferably, the planar substrate 49 is fabricated from a polymer, glass or silicon-based material, for example a microscope slide, and most preferably it is in the form of a microarray. Thereafter, the measuring unit 35 arranged to perform conventional fluorescence microscopy is used to analyse the support-plated substrate 49 systematically. Preferred paths 60 for systematically interrogating the substrate 49 are shown in Figure 6a and 6b. Figure 6a is a depiction of a meander-type interrogation regime, whereas Figure 6b is a depiction of a spiral-type interrogation regime. There are of course many other possible paths 60 apparent to one skilled in the art, for example moving the substrate 49 in an opposite direction to the path 60, or moving the substrate in a meandering diagonal path. However, the regimes of Figures 6a, 6b are efficient for achieving an enhanced support 1 read speed. Preferably, a stepper-motor actuated base plate 50 supporting and bearing the substrate 49 is used to move the substrate 49 around while the measuring unit 35 is held stationary. The positions of supports 1 are tracked so that they are analysed once only.

The planar measuring unit's 35 reader unit 37 for image-processing is used to capture digital images of each field of the substrate 49 to which supports 1 have become affixed. Digital images thereby obtained correspond to light transmitted through the substrate 49 and base plate 50 and then through the supports 1 rendering the supports 1 in silhouette view; such silhouette images of the supports 1 are analysed by the reader unit 37 in combination with a processing unit 55. The bar code 2 associated with each support 1 is hence identified from its transmitted light profile by the reader unit 37. The signal emitting unit 39 generates a fluorescent signal, which signal makes the labels 9 on supports 1 that have interacted with the protein characteristic molecules 8 fluoresce 10. A detector unit 40 detects the magnitude of fluorescence 10 from activated supports 1 to identify the degree of reaction. The fluorescent signal 10 integrated over activated supports' 1 surface 11 is recorded in association with the identification bar-code 2 to construct data sets susceptible to statistical analysis.

The processing unit 55 is connected to the light source 38, the signal unit 39, the reader unit 37, and the detector unit 40 and to a display 56. Moreover, the processing unit 55 comprises a control system for controlling the light source 38 and the signal unit 39. The light silhouette and fluorescent signals 10 from the supports 1 pass via an optical assembly 51, for example an assembly comprising one or more lenses and/or one or more mirrors, towards the detector unit 40 and reader unit 37. A mirror 52 is used to divide the optical signals into two paths and optical filters 53, 54 are used to filter out unwanted optical signals based on their wavelength. Alternatively, the light source 38 and signal unit 39 can be turned on and off at intervals, for example mutually alternately. Signals are received from the reader unit 37 and detector unit 40, which are processed and corresponding statistical analysis results presented on a display 56. Similar numbers of each type of supports 1 are required to give optimal statistical analysis of experiments. Such statistical analysis is well known in the art.

The preferred embodiment of the biochemical method of detecting one or more protein characteristics utilises the supports 1 with bar codes 2 described previously. The method comprises several steps, which can be performed in several different orders, and will now be described in more detail.

Information molecules 7 are attached to at least a main surface 11 of the supports 1 to allow the detection of potential protein characteristic matter 8 in a sample. Supports 1 with at least one type of bar code 2 are then suspended in a fluid 6 to allow a 3-dimensional array where the supports 1 are submersed in the fluid 6. The 3-dimensional array allows for very good reaction kinetics. The number of different types of supports I suspended in the fluid 6 is dependant on the test throughput required, but could be hundreds, thousands or even millions. The number of the same types of supports 1 suspended in the fluid 6 is amongst other things dependent on quality of statistical analysis and the ease of analysis.

The sample, potentially containing protein characteristic matter 8; to be analysed is added to the fluid 6 before or after the supports 1 have been suspended in the fluid. Signal emitting labels 9 are also added to the fluid 6. These signal emitting labels 9 are used to indicate interaction, e.g. bonding, between the information molecules 7 on the supports 1 and the protein characteristic matter 8 sought in the analysed sample. There are many different ways of adding the signal emitting labels 9 to the fluid 6. They can, for example, be added to the fluid 6 separately, be attached to the protein characteristic matter 8 to be analysed prior to the sample being added to the fluid 6, or be attached to the information molecule 7 before or after their attachment to the supports 1. There are also many different ways for the signal emitting labels 9 to indicate that interaction between the information molecules 7 and the protein characteristic matter 8 in the analysed sample,

One such way is for a signal, such as fluorescence or light of other wavelength (colour), to be activated by the signal emitting label 9 if there is interaction between an information molecule 7, a matching protein characteristic matter 8 and the signal emitting label 9. Alternatively the signal emitting labels 9 are activated before any interaction with the protein characteristic matter 8. When there is an interaction between the information molecule 7 and the protein characteristic matter 8 the active signal emitting label 9 is released from the other molecules deactivating its signal. This would result in a detection that is opposite to the ones discussed previously, i.e. the absence of a signal indicates that a reaction has occurred on a support in e.g. a yes/no experiment Similarly a decrease in the fluorescent signal 10 can be an indicator of the amount of protein characteristic matter 8 present in the analysed sample introduced into the fluid 6.

The fluid 6 containing supports 1 with information molecules 7, the sample to be analysed 8 and the signal emitting labels 9 is analysed using a detecting unit 40 and a reader unit 37. The reader unit 37 reads the bar code 2 of at least those supports 1 with information molecules 7 that have reacted with the protein characteristic matter 8 in the analysed sample. It may also be preferred to read the bar codes 2 of all the supports 1 as a quality control of the multiplexed experiment. The detection unit 40 detects the absence or presence of interaction signals 10 of the signal emitting labels 9. In an alternative type of biochemical assay method more than one signal may be used on each support indicating the presence of two or more protein characteristics 8 in the analysed sample. This would mean that two or more different information molecules 7 were attached to the same support 1. In such a case the signal emitting labels 9 would give off a different signal 10 depending on the protein characteristic matter 8 bonding to the information molecules 7. Another preferred methodology used for the detection of protein characteristics is to use the combined signal from two or more supports 1 with different bar codes 2 to indicate the presence of the protein characteristic. The signal combinations could, for example, be an active support A and passive support B, active supports A and B, or a passive support B and active support A, each different combination of supports indicating what type of protein characteristic is detected in the fluid.

The intended uses of the biochemical assay for detecting one or more protein characteristic includes drug targeting, proteomics or analysis of analytes. These uses of the bioassay methods are also suitable for use in the field of screening and diagnostics.

It will be appreciated that modifications can be made to embodiments of the invention described in the foregoing without departing from the scope of the invention as defined by the appended claims.

## Claims

1. A biochemical method of detecting one or more protein characteristics, the method utilizing supports (1) whose largest dimension (3) is less than 250 µm and wherein each support (1) incorporates sequential identification means (2), the method comprising the steps of:
(a) attaching an information molecule (7) to a main surface (11) of each support (1);
(b) suspending the supports (1) with associated information molecule (7) in a fluid;
(c) adding a sample (8) to be analysed to the fluid;
(d) placing the fluid comprising its associated supports (1) and sample (8) on a substrate (49) for subsequent interrogation ;
(e) detecting interaction signals from at least one of the supports (1) in the fluid using signal detecting means (40); and
(f) reading the sequential identification means (2) of the supports (1) which have an interaction signal using reading means (3), thereby detecting at least one of said one or more protein characteristics (8),
**characterised in that**
(g) the information molecule (7) is capable of interacting with at least one of said one or more protein characteristics; and
(h) the sequential identification means (2) is a bar code and integrally incorporates at least one of spatial orientation features and error correction features for assisting the reading means (3) to determine identities of the supports (1).

2. A method according to Claim 1, **characterised in that** the method further includes a step of oxidizing the supports (1) prior to attachment of associated information molecules (7) thereto.

3. A method according to Claim 1 or 2, **characterised in that** either one of the supports (1) with associated information molecules and sample (8) is added to the fluid either before, after or simultaneously as the fluid is placed on the substrate (49), **in that** step (c) is performed either before, after or simultaneously with step (b), and that step (f) is performed either before or after step (d).

4. A method according to Claim 1, 2 or 3, **characterised in that** the method further includes a step of using a measuring unit (35) for detecting the interaction signals and for reading the bar codes (2), the measuring unit (35) being arranged to detect the interaction signals and for reading of the bar codes (2) substantially simultaneously, the measuring unit (35) comprising the detecting and reading means (37, 40) for interrogating the supports (1).

5. A method according to any one or more of Claims 1 to 4, **characterised in that** signal emitting labels (9) are added to the fluid, said emitting labels (9) arranged to emit the interaction signals when information molecules (7) bind to molecules in the sample exhibiting one or more protein characteristics.

6. A method according to any one or more of Claims 1 to 5, **characterised in that** the method further comprises a step of reading the fluid along a predetermined path (60) along the substrate (49) using a measuring unit (35) arranged for detecting and reading the supports (1), said path (60) arranged to receive substantially all of the fluid.

7. A method to any one or more of Claims 1 to 6, **characterised in that** the method further comprises a step of interrogating individual supports (1) only once.

8. A method according to any one or more of Claims 1 to 5, **characterised in that** the fluid, said fluid comprising the supports (1) with associated information molecules (7) and the sample including molecules susceptible to exhibiting at least one potential protein characteristic (8), is passed through an interrogation zone (34) of a measuring unit (35) via focusing means for aligning and separating the supports (1) prior to interrogation.

9. A method according to any one or more of Claims 1 to 8, **characterised in that** a measuring unit (35) verifies reading and detection information from the supports (1) with a database including preset information linking specific bar codes (2) to specific information molecules (7).

10. A method according to any one of Claims 1 to 9, **characterised in that** the supports (1) are coated with a binding promoter on one or more of their main surfaces (11) to facilitate molecule attachment thereto.

11. A method according to Claim 10, **characterised in that** the binding promoter is at least one of silane and avidin-biotin.

12. A method according to any one of more of Claims 1 to 11, **characterised in that** the fluid includes a liquid solution.

13. A method according to any one or more of Claims 1 to 12, **characterised in that** one or more protein characteristics being detected is drug targeting and the specific types of information molecules (7) being attached to the supports (1) are nucleic acid, protein and/or PNA molecules.

14. A method according to any one or more of Claims 1 to 12, **characterised in that** one or more protein characteristics being detected is proteomics and the specific types of information molecules (7) being attached to the supports (1) are nucleic acid, protein and/or PNA molecules.

15. A method according to any one or more of Claims 1 to 12, **characterised in that** one or more protein characteristics being detected is the analysis of analytes and the specific types of information molecules (7) being attached to the supports (1) are enzyme, protein and/or PNA molecules.

16. A protein characteristic detection apparatus for analysing a fluid comprising supports (1) whose largest dimension is less than 250 µm and wherein each support (1) incorporates sequential identification means (2), the apparatus including a substrate (49) for placement of the fluid comprising supports (1) thereon for subsequent interrogation, the apparatus further including detecting means (40) and reading means (37) for detecting a plurality of independent signals generated from each support (1) when interrogated in the apparatus, at least the reading means (37) being arranged to be in optical communication with the supports (1) suspended in the fluid for detecting the sequential identification means (2) of the supports (1), and the detecting means (40) being arranged to detect an interaction signal for detection of interaction between one or more molecules exhibiting protein characteristics in a sample to be analysed and an information molecule (7) attached to a main surface (11) of supports (1) including a corresponding specific sequential identification means (2), **characterised in that** the sequential identification means is a bar code and integrally incorporates at least one of spatial orientation features and error correction features for assisting the reading means (37) to determine identities of the supports (1).

17. An apparatus according to Claim 16, **characterised in that** the interaction signal is generated by a signal emitting label (9) susceptible to being activated and deactivated through interaction between the information molecule (7) and said one or more molecules susceptible to exhibiting protein characteristics (8) in the analysed sample.

## Patentansprüche

1. Biochemisches Verfahren zur Detektion einer oder mehrerer Proteineigenschaften, worin das Verfahren Träger (1) verwendet, deren größte Dimension (3) weniger als 250 µm beträgt, und worin jeder Träger (1) sequenzielle Identifikationsmittel umfasst (2), wobei das Verfahren folgende Schritte umfasst:
(a) Anbringen eines Informationsmoleküls (7) an einer Hauptoberfläche (11) jedes Trägers (1);
(b) Suspendieren der Träger (1) mit dem assoziierten Informationsmolekül (7) in einem Fluid;
(c) Zugeben einer zu analysierenden Probe (8) zum Fluid;
(d) Positionieren des Fluids mit den assoziierten Trägern (1) und von Probe (8) auf einem Substrat (49), zur darauf folgenden Abfrage;
(e) Detektion von Wechselwirkungssignalen von zumindest einem der Träger (1) im Fluid mithilfe von Signaldetektionsmitteln (40); und
(f) Ablesen der sequenziellen Identifikationsmittel (2) der Träger (1), die ein Wechselwirkungssignal aussenden, unter Verwendung von Ablesemitteln (3), wodurch zumindest eine dieser ein oder mehreren Proteineigenschaften (8) detektiert wird,
**dadurch gekennzeichnet, dass**
(g) das Informationsmolekül (7) mit zumindest einer dieser ein oder mehreren Proteineigenschaften wechselwirken kann; und
(h) das sequenzielle Identifikationsmittel ein Barcode (2) ist und zumindest eines von räumlichen Orientierungsmerkmalen und Fehlerkorrekturmerkmalen zur Unterstützung der Ablesemittel (3) zur Bestimmung der Identitäten der Träger (1) darin integriert aufweist.

2. Verfahren nach Anspruch 1, **dadurch gekennzeichnet, dass** das Verfahren weiters einen Schritt des Oxidierens der Träger (1) vor Anbringung der damit assoziierten Informationsmoleküle (7) umfasst.

3. Verfahren nach Anspruch 1 oder 2, **dadurch gekennzeichnet, dass** entweder einer der Träger (1) mit den assoziierten Informationsmolekülen oder Probe (8) dem Fluid entweder vor, nach oder gleichzeitig mit dem Positionieren des Fluids auf dem Substrat (49) hinzugefügt wird, **dadurch**, dass Schritt (c) entweder vor, nach oder gleichzeitig mit Schritt (b) durchgeführt wird und **dadurch**, dass Schritt (f) vor oder nach Schritt (d) durchgeführt wird.

4. Verfahren nach Anspruch 1, 2 oder 3, **dadurch gekennzeichnet, dass** das Verfahren weiters einen Schritt des Verwendens einer Messeinheit (35) zur Detektion der Wechselwirkungssignale und zum Ablesen der Barcodes (2) umfasst, worin die Messeinheit (35) so angeordnet wird, dass sie im Wesentlichen gleichzeitig die Wechselwirkungssignale detektiert und die Barcodes (2) abliest, wobei die Messeinheit (35) die Detektions- und Ablesemittel (37, 40) zum Abfragen der Träger (1) umfasst.

5. Verfahren nach einem oder mehreren der Ansprüche 1 bis 4, **dadurch gekennzeichnet, dass** signalaussendende Markierungen (9) dem Fluid zugesetzt werden, wobei diese Markierungen (9) so angeordnet werden, dass sie die Wechselwirkungssignale aussenden, wenn sich die Informationsmoleküle (7) an Moleküle in der Probe binden, die eine oder mehrere Proteineigenschaften aufweisen.

6. Verfahren nach einem der Ansprüche 1 bis 5, **dadurch gekennzeichnet, dass** das Verfahren weiters einen Schritt des Ablesens des Fluids entlang eines vorgegebenen Wegs (60) entlang des Substrats (49) unter Verwendung einer Messeinheit (35) umfasst, die so angeordnet wird, dass sie die Träger (1) detektiert und abliest, wobei dieser Weg (60) so angelegt ist, dass im Wesentlichen das gesamte Fluid aufgenommen wird.

7. Verfahren nach einem oder mehreren der Ansprüche 1 bis 6, **dadurch gekennzeichnet, dass** das Verfahren weiters einen Schritt des lediglich einmaligen Abfragens einzelner Träger (1) umfasst.

8. Verfahren nach einem oder mehreren der Ansprüche 1 bis 5, **dadurch gekennzeichnet, dass** das Fluid, wobei das Fluid die Träger (1) mit assoziierten Informationsmolekülen (7) umfasst und die Probe Moleküle umfasst, die zumindest eine potenzielle Proteineigenschaft (8) aufweisen, mittels Fokussierungsmitteln zur Anordnung und Trennung von Trägern (1) vor der Abfrage durch eine Abfragezone (34) einer Messeinheit (35) geleitet wird.

9. Verfahren nach einem oder mehreren der Ansprüche 1 bis 8, **dadurch gekennzeichnet, dass** eine Messeinheit (35) Ablese- und Detektionsinformationen von den Trägern (12) mittels einer Datenbank mit voreingestellten Informationen verifiziert, die spezifische Barcodes (2) mit spezifischen Informationsmolekülen (7) verknüpfen.

10. Verfahren nach einem der Ansprüche 1 bis 9, **dadurch gekennzeichnet, dass** die Träger (1) auf einer oder mehreren ihrer Hauptoberflächen (11) mit einem Bindungspromotor überzogen sind, um die Molekülanbringung daran zu erleichtern.

11. Verfahren nach Anspruch 10, **dadurch gekennzeichnet, dass** der Bindungspromotor zumindest eines von Silan oder Avidin-Biotin ist.

12. Verfahren nach einem der Ansprüche 1 bis 11, **dadurch gekennzeichnet, dass** das Fluid eine flüssige Lösung umfasst.

13. Verfahren nach einem oder mehreren der Ansprüche 1 bis 12, **dadurch gekennzeichnet, dass** eine oder mehrere der detektierten Proteineigenschaften Arzneimitteltargeting ist und die spezifischen Typen der Informationsmoleküle (7), die an den Trägern angebracht sind, Nucleinsäure-, Protein- und/oder PNA-Moleküle sind.

14. Verfahren nach einem oder mehreren der Ansprüche 1 bis 12, **dadurch** gekenn-zeichnet, dass eine oder mehrere der detektierten Proteineigenschaften Proteomik ist und die spezifischen Typen der Informationsmoleküle (7), die an den Trägern angebracht sind, Nucleinsäure-, Protein- und/oder PNA-Moleküle sind.

15. Verfahren nach einem oder mehreren der Ansprüche 1 bis 12, **dadurch gekennzeichnet, dass** eine oder mehrere der detektierten Proteineigenschaften die Analyse von Analyten ist und die spezifischen Typen der Informationsmoleküle (7), die an den Trägern angebracht sind, Enzym-, Protein- und/oder PNA-Moleküle sind.

16. Proteineigenschaftsdetektionsgerät zur Analyse eines Fluids, das Träger (1) umfasst, deren größte Dimension weniger als 250 µm beträgt, und worin jeder Träger (1) sequenzielle Identifikationsmittel (2) umfasst, wobei das Gerät ein Substrat (49) zur Positionierung des Träger (1) umfassenden Fluids darauf zur darauf folgenden Abfrage umfasst, wobei das Gerät weiters Detektionsmittel (40) und Ablesemittel (37) zur Detektion einer Vielzahl unabhängiger Signale umfasst, die von jedem Träger (1) erzeugt werden, wenn dieser im Gerät abgefragt wird, worin zumindest die Ablesemittel (37) so angeordnet sind, dass sie in optischer Verbindung mit den im Fluid suspendierten Trägern (1) stehen, um die sequenziellen Identifikationsmittel (2) der Träger (1) zu detektieren, und die Detektionsmittel so angeordnet sind, dass sie ein Wechselwirkungssignal detektieren, um Wechselwirkung zwischen einem oder mehreren Molekülen mit Proteineigenschaften in einer zu analysierenden Probe und einem Informationsmolekül (7) zu detektieren, das an einer Hauptoberfläche (11) von Trägern (1) angebracht ist, die ein entsprechendes spezifisches sequenzielles Identifikationsmittel (2) umfassen, **dadurch gekennzeichnet, dass** das sequenzielle Identifikationsmittel ein Barcode ist und zumindest eines von räumlichen Orientierungsmerkmalen und Fehlerkorrekturmerkmalen darin integriert aufweist, um die Ablesemittel (37) zu unterstützen, um die Identität der Träger (1) zu bestimmen.

17. Gerät nach Anspruch 16, dass **dadurch gekennzeichnet ist, dass** das Wechselwirkungssignal von einer signalaussendenden Markierung (9) erzeugt wird, die durch Wechselwirkung zwischen dem Informationsmolekül (7) und dem einen oder den mehreren Molekülen in der analysierten Probe, die Proteineigenschaften zeigen können, aktiviert und deaktiviert werden kann.

## Revendications

1. Procédé biochimique de détection d'une ou plusieurs caractéristiques de protéine, le procédé utilisant des supports (1) dont la dimension la plus grande (3) est inférieure à 250 µm et dans lequel chaque support (1) incorpore un moyen d'identification séquentiel (2), ledit procédé comprenant les étapes consistant à :
(a) fixer une molécule d'information (7) à une surface principale (11) de chaque support (1) ;
(b) mettre en suspension les supports (1) avec les molécules d'information (7) dans un fluide ;
(c) ajouter au fluide un échantillon (8) à analyser ;
(d) placer le fluide comprenant ses supports (1) associés et l'échantillon (8) sur un substrat (49) pour interrogation ultérieure ;
(e) détecter les signaux d'interaction d'au moins l'un des supports (1) dans le fluide en utilisant un moyen (40) de détection de signal ; et
(f) lire le moyen d'identification séquentiel (2) des supports (1) qui ont un signal d'interaction en utilisant le moyen de lecture (3), détectant ainsi au moins l'une desdites une ou plusieurs caractéristiques (8) de protéine,
**caractérisé en ce que**
(g) la molécule d'information (7) est capable d'interagir avec au moins l'une desdites une ou plusieurs caractéristiques de protéine et ;
(h) le moyen d'identification séquentiel (2) est un code à barres et incorpore intégralement au moins une caractéristique d'orientation spatiale ou caractéristique de correction d'erreur pour aider le moyen de lecture (3) à déterminer les identités des supports (1).

2. Procédé selon la revendication 1, **caractérisé en ce que** le procédé inclut en outre une étape consistant à oxyder les supports (1) avant fixation de molécules d'information (7) associées à ces supports.

3. Procédé selon la revendication 1 ou 2, **caractérisé en ce que** l'un des supports (1) avec les molécules d'information associées et un échantillon (8) est ajouté au fluide avant, après ou pendant le placement du fluide sur le substrat (49), **en ce que** l'étape (c) est réalisée avant, après ou pendant l'étape (b) et **en ce que** l'étape (f) est réalisée avant ou après l'étape (d).

4. Procédé selon la revendication 1, 2 ou 3, **caractérisé en ce que** le procédé inclut en outre une étape consistant à utiliser une unité de mesure (35) pour détecter les signaux d'interaction et pour lire les codes à barres (2), l'unité de mesure (35) étant arrangée pour détecter les signaux d'interaction et pour lire les codes à barres (2) sensiblement simultanément, l'unité de mesure (35) comprenant les moyens de détection et de lecture (37, 40) pour interroger les supports (1).

5. Procédé selon l'une quelconque des revendications 1 à 4, **caractérisé en ce que** des marqueurs émetteurs de signal (9) sont ajoutés au fluide, lesdits marqueurs émetteurs (9) étant arrangés pour émettre les signaux d'interaction lorsque les molécules d'information (7) se lient à des molécules dans l'échantillon présentant une ou plusieurs caractéristiques de protéine.

6. Procédé selon une ou plusieurs quelconques des revendications 1 à 5, **caractérisé en ce que** le procédé comprend en outre une étape consistant à lire le fluide le long d'un trajet prédéterminé (60) le long du substrat (49) en utilisant une unité de mesure (35) arrangée pour détecter et lire les supports (1), ledit trajet (60) étant arrangé pour recevoir sensiblement la totalité du fluide.

7. Procédé selon une ou plusieurs quelconques des revendications 1 à 6, **caractérisé en ce que** le procédé comprend en outre une étape consistant à interroger les supports (1) individuels une seule fois.

8. Procédé selon une ou plusieurs quelconques des revendications 1 à 5, **caractérisé en ce que** le fluide, ledit fluide comprenant les supports (1) avec les molécules d'information (7) associées et l'échantillon incluant les molécules susceptibles de présenter au moins une caractéristique de protéine potentielle (8), passent à travers une zone d'interrogation (34) d'une unité de mesure (35) via un moyen de focalisation pour aligner et séparer les supports (1) avant interrogation.

9. Procédé selon l'une quelconque des revendications 1 à 8, **caractérisé en ce qu'**une unité de mesure (35) vérifie les informations de lecture et de détection des supports (1) avec une base de données incluant des informations préétablies reliant des codes à barres spécifiques (2) à des molécules d'information spécifiques (7).

10. Procédé selon l'une quelconque des revendications 1 à 9, **caractérisé en ce que** les supports (1) sont revêtus d'un promoteur de liaison sur une ou plusieurs de leurs surfaces principales (11) pour faciliter la fixation de molécules.

11. Procédé selon la revendication 10, **caractérisé en ce que** le promoteur de liaison est au moins le silane ou l'avidine - biotine.

12. Procédé selon une ou plusieurs quelconques des revendications 1 à 11, **caractérisé en ce que** le fluide inclut une solution liquide.

13. Procédé selon une ou plusieurs quelconques des revendications 1 à 12, **caractérisé en ce qu'**une ou plusieurs caractéristiques de protéine détectées sont le ciblage de médicament et les types spécifiques de molécules d'information (7) fixées aux supports (1) sont des molécules d'acide nucléique, de protéine et/ou de PNA.

14. Procédé selon une ou plusieurs quelconques des revendications 1 à 2, **caractérisé en ce qu'**une ou plusieurs caractéristiques de protéine détectées sont protéomiques et **en ce que** les types spécifiques de molécules d'information (7) fixées aux supports (1) sont des molécules d'acide nucléique, de protéine et/ou de PNA.

15. Procédé selon une ou plusieurs quelconques des revendications 1 à 12, **caractérisé en ce qu'**une ou plusieurs caractéristiques de protéine détectées sont l'analyse d'analytes et **en ce que** les types spécifiques de molécules d'information (7) fixées aux supports (1) sont des molécules d'acide nucléique, de protéine et/ou de PNA.

16. Appareil de détection de caractéristique de protéine pour analyser un fluide comprenant des supports (1) dont la dimension la plus grande est inférieure à 250 *µ*m et dans lequel chaque support (1) incorpore un moyen d'identification séquentielle (2), l'appareil comprenant un substrat (49) pour placement des supports (1) comprenant un fluide pour interrogation ultérieure, l'appareil incluant en outre un moyen de détection (40) et un moyen de lecture (37) pour détecter une pluralité de signaux indépendants générés par chaque support (1) interrogé dans l'appareil, au moins le moyen de lecture (37) étant arrangé pour être en communication optique avec les supports (1) en suspension dans le fluide pour détecter le moyen d'identification séquentielle (2) des supports (1), et le moyen de détection (40) étant arrangé pour détecter un signal d'interaction pour la détection de l'interaction entre une ou plusieurs molécules présentant des caractéristiques de protéine dans un échantillon à analyser et une molécule d'interaction (7) fixée à une surface principale (11) des supports (1) incluant un moyen d'identification séquentiel (2) correspondant spécifique, **caractérisé en ce que** le moyen d'identification séquentiel est un code à barres et incorpore intégralement au moins une caractéristique d'orientation spatiale ou caractéristique de correction d'erreur pour aider le moyen de lecture (37) à déterminer les identités des supports (1).

17. Appareil selon la revendication 16, **caractérisé en ce que** le signal d'interaction est généré par un marqueur émetteur de signal (9) susceptible d'être activé et désactivé par interaction entre la molécule d'information (7) et lesdites une ou plusieurs molécules susceptibles de présenter des caractéristiques (8) de protéine dans l'échantillon analysé.
